# EUROPEAN PATENT APPLICATION

(11) **EP 2 259 058 A1**
(43) Date of publication of application: **08.12.2010**
(21) Application number: 09726358.6
(22) Date of filing: 20.02.2009
(51) Int. Cl.: G01N 33/49, G01N 21/78, G01N 33/483, G01N 33/72

(54) **METHOD OF MEASURING HEMATOCRIT VALUE OR BLOOD COMPONENT CONCENTRATION AND MEASUREMENT SYSTEM**

(30) Priority: 25.03.2008 JP 2008078880
(71) Applicant: Terumo Kabushiki Kaisha, Shibuya-ku Tokyo 151-0072 (JP)
(72) Inventor: SUGAWARA, Yoshihisa, Ashigarakami-gun Kanagawa 259-0151 (JP)
(74) Representative: TBK-Patent
(86) International application number: PCT/JP2009/053036
(87) International publication number: WO 2009/119213

(57) **Abstract**

A method of measuring hematocrit value and a device for measuring hematocrit value which can measure hematocrit value accurately are provided.

A method of measuring hematocrit value of a blood sample in a chromogenic reaction using a chromogenic reagent that reacts with a blood component that is different from hemoglobin, **characterized by**: calculating the hematocrit value of the blood sample based on an optical characteristic a₁ of the blood sample measured with a light λ₁ of at least one absorption wavelength specific to hemoglobin and an optical characteristic a₂ of the blood sample measured with a light λ₂ of at least one absorption wavelength specific to a pigment generated in the chromogenic reaction.

## Description

### Technical field

The present invention relates to a method of measuring hematocrit value, a device for measuring hematocrit value, a method of measuring a component, and a device for measuring a component.

### Background art

It is important for the prevention and treatment of diabetes to know the blood glucose level.

As a technology for optically measuring the blood glucose level of the withdrawn blood, a determination method of the glucose concentration disclosed in the Japanese Patent Laid-Open No. 2000-262298 is known. The determination method disclosed in the Japanese Patent Laid-Open No. 2000-262298 is designed to measure a chromogenic density of a blood sample by using a light of a certain wavelength specified within a range of wavelengths shorter than 500 nm or a range of wavelengths longer than 590 nm, while measuring an optical density of red color of the pigment of erythrocyte by using a light of a certain wavelength specified within a range of 500-590 nm, employing a multilayer analytical element containing color-producing compositions. According to such a determination method, the glucose concentration can be quantified by compensating the glucose value obtained from the chromogenic density with the hematocrit value obtained from the optical density of red color.

### Disclosure of the invention

### Problems to be solved by the invention

However, the aforementioned determination method has a problem that it cannot quantify glucose concentration accurately because the measuring accuracy of the hematocrit value obtained by using a light with a certain wavelength specified within the range of wavelengths of 500-590 nm is not enough.

Also, if the wavelength used for measuring the hematocrit value overlaps with the absorption wavelength of the pigment generated by the reaction between the blood component and a chromogenic reagent, the accuracy of the hematocrit value based on the particular wavelength reduces under the influence of the overlapping of the absorption wavelength of the pigment.

The present invention is made in order to solve the aforementioned problems. Therefore, one object of the present invention is to provide a method of measuring hematocrit value and a device for measuring hematocrit value which can measure hematocrit value accurately.

Also, another object of the present invention is to provide a method of measuring a component and a device for measuring a component using the aforementioned method of measuring hematocrit value and device for measuring hematocrit value.

### Means of solving the problems

The aforementioned obj ects of the present invention can be accomplished by the inventions described in (1) through (5) below.

(1) A method of measuring hematocrit value of a blood sample in a chromogenic reaction using a chromogenic reagent that reacts with a blood component that is different from hemoglobin,characterized by:calculatingthe hematocrit value of said blood sample based on an optical characteristic of said blood sample measured with a light of at least one absorption wavelength specific to hemoglobin and an optical characteristic of said blood sample measured with a light of at least one absorption wavelength specific to a pigment generated in said chromogenic reaction.

(2) The method of measuring hematocrit value according to (1), **characterized in that** said optical characteristic of the blood sample relative to said light of the absorption wavelength of hemoglobin is measured at an earlier time point than said optical characteristic of the blood sample relative to said light of the absorption wavelength of said pigment.

(3) A method of measuring concentration of a blood component that is different from hemoglobin by means of a chromogenic reaction using a chromogenic reagent that reacts with said blood component, characterized by comprising: a step of irradiating a blood sample with a light of at least one absorption wavelength specific to hemoglobin to measure a first optical characteristic of said blood sample relative to said light of the absorption wavelength of hemoglobin; a step of irradiating said blood sample with a light of at least one absorption wavelength specific to a pigment generated in said chromogenic reaction to measure a second optical characteristic of said blood sample relative to said light of the absorption wavelength of said pigment; a step of calculating the hematocrit value of said blood sample based on said first optical characteristic and said second optical characteristic; and a step of calculating the concentration of said component of said blood sample based on said calculated hematocrit value and said second optical characteristic.

(4) A device for measuring hematocrit value of a blood sample in a chromogenic reaction using a chromogenic reagent that reacts with a blood component that is different from hemoglobin, characterized by: calculating the hematocrit value of said blood sample based on an optical characteristic of said blood sample measured with a light of at least one absorption wavelength specific to hemoglobin and an optical characteristic of said blood sample measured with a light of at least one absorption wavelength specific to a pigment generated in said chromogenic reaction.

(5) A device for measuring concentration of a blood component that is different from hemoglobin by means of a chromogenic reaction using a chromogenic reagent that reacts with said blood component, characterized by comprising: first light emitting means for irradiating a blood sample with a light of at least one absorption wavelength specific to hemoglobin; first light receiving means for receiving the light that is radiated from said first light emitting means and is reflected by said blood sample or passes through said blood sample to measure a first optical characteristic of said blood sample relative to said light of the absorption wavelength of hemoglobin; second light emitting means for irradiating said blood sample with a light of at least one absorption wavelength specific to a pigment generated in said chromogenic reaction; second light receiving means for receiving the light that is radiated from said second light emitting means and is reflected by said blood sample or passes through said blood sample to measure a second optical characteristic of said blood sample relative to said light of the absorption wavelength of said pigment; hematocrit value calculation means for calculating the hematocrit value of said blood sample based on said first optical characteristic and said second optical characteristic; and component concentration calculation means for calculating the concentration of said component of said blood sample based on said calculated hematocrit value and said second optical characteristic.

### Effect of the invention

According to the invention described in (1) above, the hematocrit value can be measured accurately as the hematocrit value is calculated from the optical characteristics of a blood sample for two kinds of light of different wavelengths.

Also, according to the invention described in (2) above, the hematocrit value can be measured with an even higher accuracy.

Also, according to the invention described in (3) above, the concentration of a blood component can be measured accurately without complicating a device configuration as the hematocrit value can be measured accurately using the light used for measuring a blood component that is different from hemoglobin.

Also, according to the invention described in (4) above, the hematocrit value can be measured accurately as the hematocrit value is calculated from the optical characteristics of a blood sample for two kinds of light of different wavelengths.

Also, according to the invention described in (5) above, the concentration of a blood component can be measured accurately without complicating a device configuration as the hematocrit value can be measured accurately using the light used for measuring a blood component that is different from hemoglobin.

### Brief description of the drawings

Fig. 1 is a perspective view of the general constitution of a component measuring device in an embodiment of the present invention.
Fig. 2 is a partial cross-section view of the component measuring device shown in Fig 1.
Fig. 3 is a block diagram showing the general constitution of the component measuring device shown in Fig. 1.
Fig. 4 is a flowchart for describing the component measurement process in the component measuring device shown in Fig. 1.
Fig. 5 is a diagram for describing the hematocrit value calculation process shown in step S110 of Fig. 4.
Fig. 6 is a diagram showing the coloring spectrum of a blood sample where glucose and hemoglobin are contained in various concentrations.
Fig. 7A is a diagram showing a chronological change of the reflection absorbance relative to a light of the absorption wavelength of a pigment of a blood sample absorbed by a test paper.
Fig. 7B is a diagram showing a chronological change of the reflection absorbance relative to a light of the absorption wavelength of a hemoglobin when a blood sample is absorbed by a test paper.
Fig. 8 is a diagram for describing the hematocrit value calculation process in a comparative example.

### Best mode for carrying out the invention

The embodiment of the present invention will be described in the following with reference to the accompanying drawings. In the embodiment described below, it is assumed that the component measuring device of the present invention is applied to a blood glucose level measuring device that measures glucose concentration in blood. The identical numeral is applied to the identical members of the drawings.

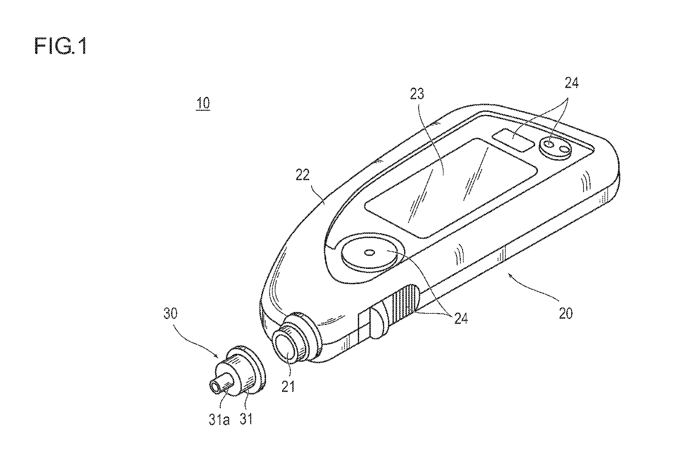

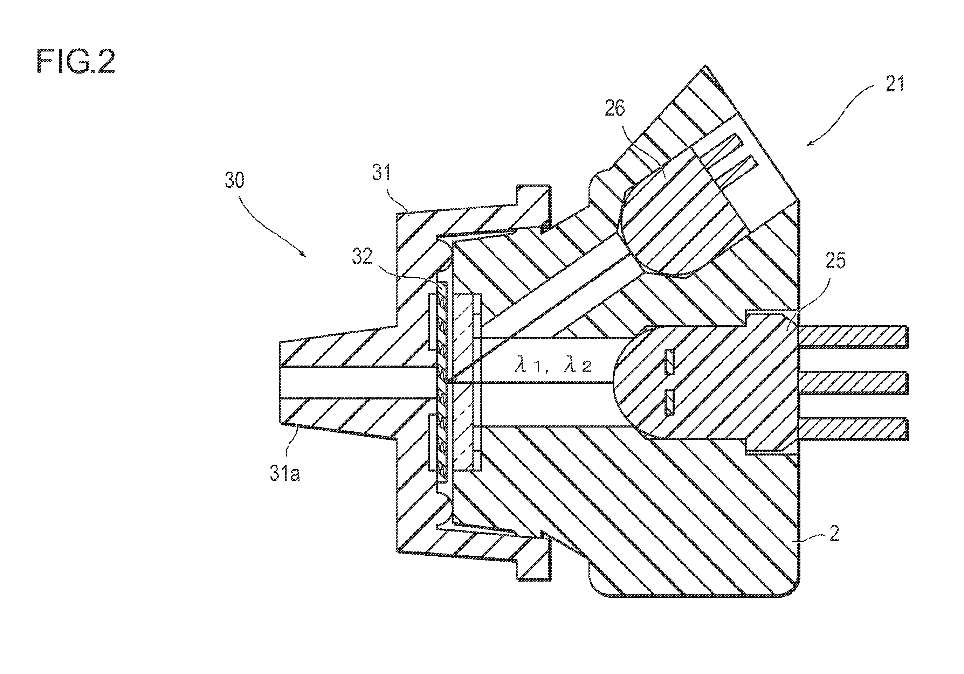

Fig. 1 is a perspective view of the general constitution of a component measuring device in an embodiment of the present invention, and Fig. 2 is a partial cross-section view of the component measuring device shown in Fig 1. The component measuring device according to the present embodiment is to calculate the hematocrit value of the blood sample by measuring the optical characteristics of the blood sample for two kinds of light of different wavelengths and to calculate the glucose concentration of a blood sample based on the calculated hematocrit value and the optical characteristic of the blood sample for one kind of light.

As shown in Fig. 1, the component measuring device 10 according to the present embodiment consists of a measuring device main body 20, and a tip 30 attached to the measuring device main body 20. Hereinafter, the tip 30 and the measuring device main body 20 will be described in that order.

### <Tip>

The tip 30 is to hold the blood sample. As shown in Fig. 1 and Fig. 2, the tip 30 has a holder 31 equipped with a fine tube part 31a and a test paper 32 fixed to the inside of the holder 31.

The fine tube part 31a introduces the blood sample into the inside of the holder 31 through its open end by capillary action. The blood sample introduced via the fine tube part 31a is absorbed by the test paper 32 inside the holder 31. The test paper 32 is composed of a sheet-like porous base material and the test paper 32 is impregnated with a chromogenic reagent that causes a chromogenic reaction reacting to glucose. With such a constitution, the blood sample introduced into the inside of the holder 31 via the fine tube part 31a causes a chromogenic reaction on the test paper 32 impregnated with the chromogenic reagent.

Polyester series, polyamide series, polyolefin series, polysulfone series, or cellulose series can be used as the material of the test paper 32. Also, glucose oxidase (GOD), peroxidase (POD), 4-aminoantipyrine, N-ethyl-N-(2-hydroxy-3-sulfopropyl)-m-toluidine sodium (TOOS) can be used as the chromogenic reagent.

### <Measuring device main body>

The measuring device main body 20 is to measure the blood glucose level of the blood sample held in the tip 30. As shown in Fig. 1, the measuring device main body 20 of the present embodiment has a light measuring unit 21 for measuring the intensity of the reflection by irradiating the blood sample with two kinds of light of different wavelengths, and a control unit (not shown) for controlling the light measuring unit 21 and executing various calculations on the light measurement data obtained by the light measuring unit 21. The light measuring unit 21 is provided at the distal end of an outer case 22. The control unit is stored inside the outer case 22 together with a power unit, etc. The details of the control unit will be described later. A display unit 23 for displaying the measurement results such as blood glucose level and the like and various switches 24 are provided on the surface of the outer case 22.

As shown in Fig. 2, the light measuring unit 21 of the present embodiment has a light emitting element 25 that emits two kinds of light λ₁ λ₂ of different wavelengths and a light receiving element 26 that receives the reflection of the two kinds of light λ₁, X₂. The light emitting element 25 and the light receiving element 26 are stored in a holder 27.

As the first and second light emitting means, the light emitting element 25 emits a light (hereinafter called the first light) λ₁ of an absorption wavelength (e.g., 545 nm) of a hemoglobin and a light (hereinafter called the second light) λ₂ of an absorption wavelength (e.g., 630 nm) of a pigment which results from the chromogenic reaction of the blood sample. The light emitting element 25 of the present invention is a light emitting diode of a dual wavelength type and controlled by the control unit to emit the first light λ₁ of the absorption wavelength of hemoglobin or the second light λ₂ of the absorption wavelength of the pigment. The first and second lights λ₁ λ₂ emitted from the light emitting element 25 of the present embodiment are pulse lights whose cycle and light-emitting time of a pulse are set for approximately 0.5 - 3.0 msec and 0.05 - 0.3 msec respectively. The first and second lights λ₁ λ₂ emitted from the light emitting element 25 are reflected by the test paper 32. Although dual wavelength type light emitting diode is used as the first and second light emitting means in this embodiment, two independent single wavelength type light emitting diodes can be used instead.

The light receiving element 26 is to measure the absorbance (optical characteristic) of the blood sample, receiving the first and second lights λ₁ λ₂ reflected by the test paper 32, as the first and second light receiving means. The light receiving element 26 of the present embodiment is a photo diode and receives the first light λ₁ of the absorption wavelength of the hemoglobin and the second light λ₂ of the absorption wavelength of the pigment, which are reflected at the test paper 32, to output their intensities as the light measurement data.

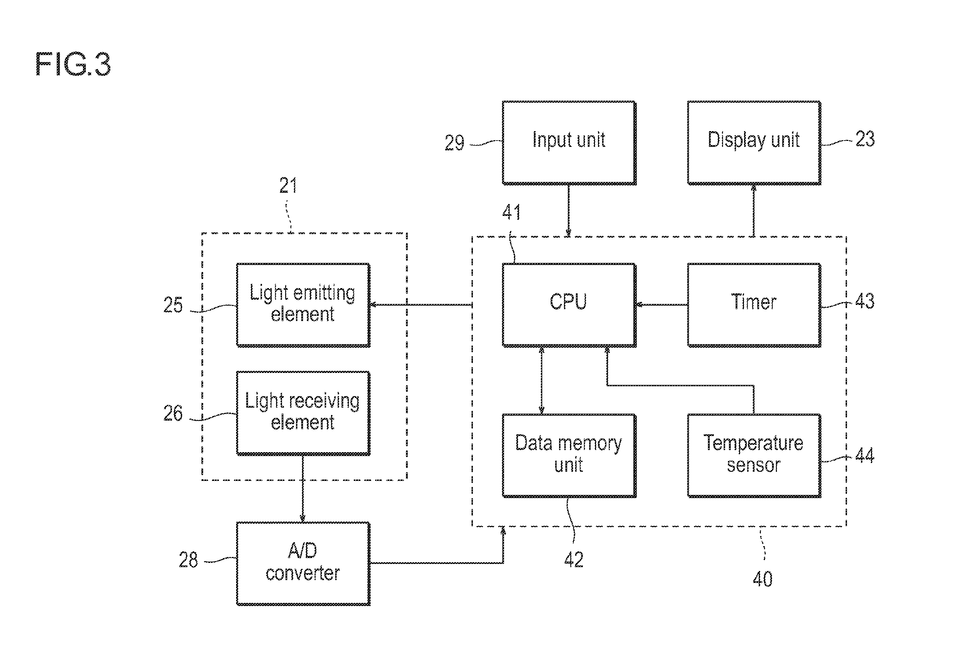

Fig. 3 is a block diagram showing the general constitution of the component measuring device shown in Fig. 1.

As mentioned above, the component measuring device 10 of the present embodiment has the light measuring unit 21 and a control unit 40. The light emitting element 25 of the light measuring unit 21 is controlled for its emission by the control unit 40, while the light measurement data from the light receiving element 26 of the light measuring unit 21 is entered into the control unit 40 via an A/D converter 28. The control unit 40 is electrically connected with the display unit 23 mounted on the outer case 22 and the input unit 29 corresponding with various switches 24.

As shown in Fig. 3, the control unit 40 of the present embodiment is equipped with a CPU 41, a data memory unit 42, a timer 43, and a temperature sensor 44. The data memory unit 42, the timer 43, and the temperature sensor 44 are electrically connected to the CPU 41 respectively.

The CPU 41 is to execute various calculations for the light measurement data with respect to the two kinds of light λ₁, λ₂ of different wavelengths. The CPU 41 of the present embodiment calculates the absorbances a₁, a₂ of the blood sample for the two kinds of light λ₁, λ₂ of different wavelengths from the light measurement data of the light receiving element 26. The CPU 41 works as the hematocrit value calculation unit (hematocrit value calculation means) as well as the component concentration calculation unit (component concentration calculation means).

The hematocrit value calculation unit calculates the hematocrit value of the blood sample based on the absorbance a₁ of the blood sample for the first light λ₁ of the absorption wavelength of the hemoglobin and the absorbance a₂ for the second light λ₂ of the absorption wavelength of the pigment generated in the chromogenic reaction of the blood sample. The component concentration calculation unit calculates the glucose concentration of the blood sample based on the hematocrit value and the absorbance a₂ for the second light λ₂. The specific process of each unit will be described later.

The data memory unit 42 is equipped with a first memory (RAM), a second memory (ROM), and a third memory (nonvolatile RAM). The first memory stores the light measurement data entered from the light measuring unit 21. The second memory stores a conversion table showing the relation between the absorbances a₁, a₂ for the first and second lights λ₁ λ₂ obtained from the light measurement data and the hematocrit value, as well as a conversion table showing the relation among the absorbance a₂ for the second light λ₂, the hematocrit value, and the glucose concentration. The second memory also stores a hematocrit value calculation program for calculating the hematocrit value of the blood sample based on the absorbance a₁ of the blood sample for the first lights λ₁ and the absorbance a₂ of the blood sample for the second light λ₂, as well as a composition concentration calculation program for calculating the glucose concentration of the blood sample based on the absorbance a₂ of the blood sample for the second light λ₂ and the hematocrit value. The third memory sores a correction value specific to each device in advance.

The timer 43 measures elapsed time and reports it to the CPU 41. The timer 43 of the present embodiment reports to the CPU 41 the first measurement time point for measuring the absorbance a₁ of the blood sample for the first light λ₁ of the absorption wavelength of the hemoglobin, and the second measurement time point for measuring the absorbance a₂ of the blood sample for the second light λ₂ of the absorption wavelength of the pigment.

The temperature sensor 44 is to measure the ambient tempeature. The temperature information obtained by the temperature sensor 44 is used for the temperature compensation calcualtion for the intended glucose concentration.

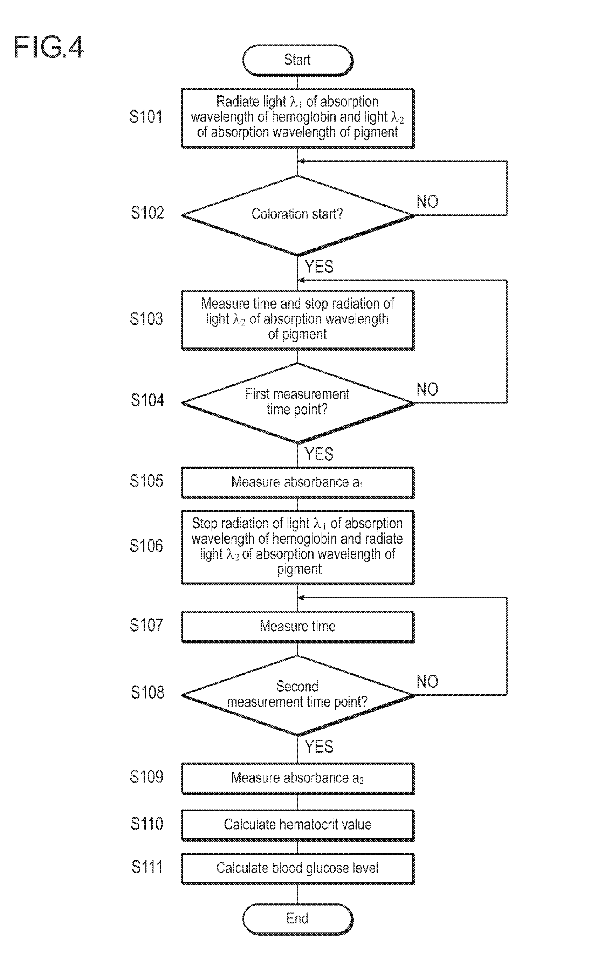

In the component measuring device 10 of the present embodiment configured as described in the above, the first light λ₁ that has the absorption wavelength specific to hemoglobin and the second light λ₂ that has the absorption wavelength specific to the pigment generated by chromogenic reaction of the blood sample are irradiated, and the absorbance a₁ of the blood sample for the first light λ₁ at the first measurement time point and the absorbance a₂ of the blood sample for the second light λ₂ at the second measurement time point, which is later than the first measurement time point, are measured. Next, the hematocrit value of the blood sample is calculated based on the two measured absorbances a₁ and a₂. The glucose concentration of the blood sample is then calculated based on the hematocrit value and the absorbance a₂ of the blood sample for the second light λ₂. The component measuring method of the present embodiment will be described below with reference to Fig. 4 through Fig. 7.

Fig. 4 is a flowchart for describing the component measurement process in the component measuring device shown in Fig. 1.

As shown in Fig. 4, in the component measurement process of the present embodiment, first, the test paper 32 is irradiated with the first light λ₁ and the second light λ₂ those are emitting alternately in a time-sharing manner in order to measure the standard value of the first light λ₁ that has the absorption wavelength specific to hemoglobin and the standard value of the second light λ₂ that has the absorption wavelength specific to the pigment, before spotting the blood sample (step S101). In the present embodiment, the second light λ₂ of the absorption wavelength of the pigment is used to detect that the blood sample is absorbed by the test paper 32 and the chromogenic reaction starts as a result. Moreover, in the composition measuring device 10 of the present embodiment, the blood sample is absorbed into the test paper 32 from one surface of the test paper 32 while the other surface of the test paper 32 is irradiated by the first light λ₁ and the second light λ₂ for detecting the coloring.

Next, a judgment is made as to whether or not coloration has started (step S102). In the present embodiment, the CPU 41 makes a judgment as to whether or not the coloration has started by detecting the variation of the absorbance a₂ per a specific time period.

If the coloration has not started (step S102: No), the process waits until the coloration starts. On the other hand, if the coloration has started (step S102: Yes), time measurement starts assuming that the chromogenic reaction by the blood sample has started (step S103). In the present embodiment, the timer 43 measures the time elapsed from the time point coloration starts. Also, the irradiation of the second light λ₂ of the absorption wavelength of the pigment is temporarily stopped at the time point when the start of coloration is detected.

Next, a judgment is made as to whether it is the first measurement time point or not (step S104). In the present embodiment, the CPU 41 makes a judgment as to whether or not it is the first measurement time point for measuring the absorbance a₁ for the first light λ₁ of the absorption wavelength of the hemoglobin by determining whether there is a report from the timer 43 or not. In this case, the first measurement time point is, for example, a time point of 5 seconds after the start of coloration. The first measurement time point is determined based on the materials and dimensions of the fine tube part 31a and the test paper 32.

If it is not yet the first measurement time point (step S104: No), the process waits until the first measurement time point is reached. On the other hand, if the first measurement time point is reached (step S104: Yes), the absorbance a₁ for the first light λ₁ at the first measurement time point is measured (step S105) . In the present embodiment, the CPU 41 calculates the absorbance a₁ of the blood sample for the first light λ₁ of the absorption wavelength of the hemoglobin at the first measurement time point from the output of the light receiving element 26 at the first measurement time point. The calculated absorbance a₁ is stored in the data memory unit 42.

Next, the second light λ₂ of the absorption wavelength of the pigment related to the chromogenic reaction of the blood sample is radiated in place of the first light λ₁ of the absorption wavelength of the hemoglobin (step S106) . In the present embodiment, the dual wavelength type light emitting element 25 emits the second light λ₂ of the absorption wavelength of the pigment in place of the first light λ₁ of the absorption wavelength of the hemoglobin. Further, in the present embodiment, although it is constituted in such a way as to radiate the first light λ₁ from the coloration start time point to the first measurement time point and to radiate the second light λ₂ from the first measurement time point to the second measurement time point, it can also be constituted in such a way as to radiate continuously the first light λ₁ and the second light λ₂ but alternately until the second measurement time point, or only radiate the second light λ₂ continuously.

Next, time is measured to determine whether it is the second measurement time point or not (step S107, S108). In the present embodiment, the CPU 41 makes a judgment as to whether or not it is the second measurement time point for measuring the absorbance a₂ for the second light λ₂ of the absorption wavelength of the pigment by determining whether there is a report from the timer 43 or not. In this case, the second measurement time point, for example, 10 seconds after the start of coloration, is a time point later than the first measurement time point. The second measurement time point is determined by the materials and dimensions of the fine tube part 31a and the test paper 32.

If it is not yet the second measurement time point (step S108: No), the process waits until the secondmeasurement time point is reached. On the other hand, if the second measurement time point is reached (step S108: Yes), the absorbance a₂ for the second light λ₂ at the second measurement time point is measured (step S109) . In the present embodiment, the CPU 41 calculates the absorbance a₂ of the blood sample for the second light λ₂ of the absorption wavelength of the pigment at the second measurement time point from the output of the light receiving element 26 at the second measurement time point. The calculated absorbance a₂ is stored in the data memory unit 42.

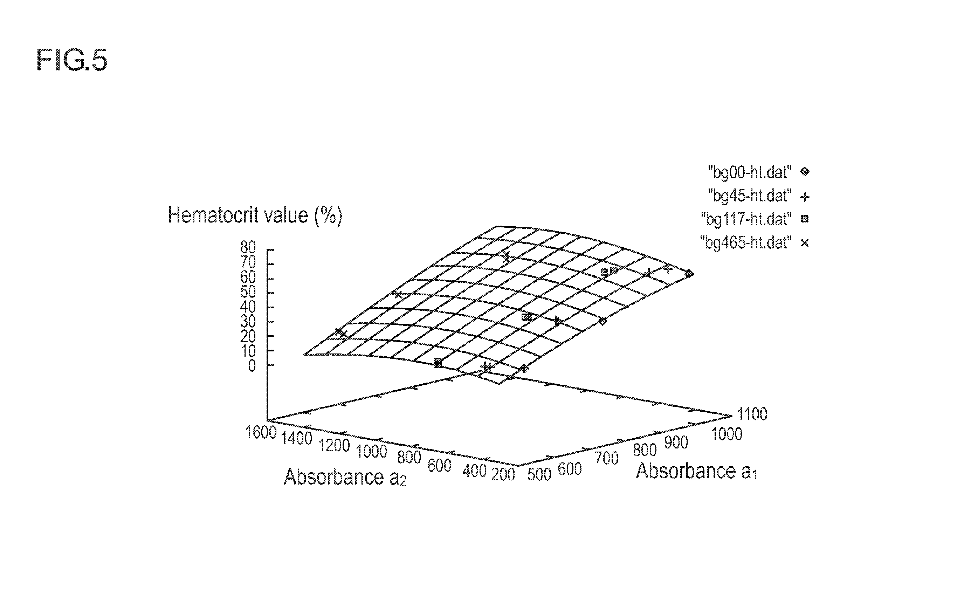

Next, the hematocrit value is calculated based on the absorbances a₁ and a₂ measured in steps S105 and S109 (step S110). In the present embodiment, the CPU 41 calculates the hematocrit value of the blood sample from the measured absorbances a₁ and a₂ based on the absorbance/hematocrit value conversion table, which is stored in the data memory unit 42 in advance, showing the relation among the absorbance a₁ for the first light λ₁, the absorbance a₂ for the second light X₂, and the hematocrit value. Further, as shown in Fig. 5, the absorbance/hematocrit value conversion table in the present embodiment is stored in the data memory unit 42 as a polynomial equation of the second order (approximate curvilinear surface) calculated by the least square method based on multiple reference data acquired in advance. The calculated hematocrit value is stored in the data memory unit 42.

Then, the glucose concentration of the blood sample is calculated (step S111). In the present embodiment, the CPU 41 calculates the glucose concentration of the blood sample based on the hematocrit value calculated in the process shown in step S110 and the the absorbance a₂ for the second light λ₂ measured in the process shown in step S109. More specifically, the CPU 41 calculates the glucose concentration based on the hematocrit value/absorbance/glucose concentration conversion table, which is stored in the data memory unit 42 in advance, showing the relation among the hematocrit value, the absorbance a₂, and the glucose concentration. The calculated glucose concentration is displayed on the display unit 23 as the blood glucose level.

As can be seen from the above, the first light λ₁ of the absorption wavelength of the hemoglobin is radiated and the absorbance a₁ of the blood sample at the first measurement time point is measured according to the process shown in the flowchart of Fig. 4. Also, the second light λ₂ of the absorption wavelength of the pigment related to chromogenic reaction of the blood sample is radiated and the absorbance a₂ of the blood sample is measured at the second measurement time point which is later than the first measurement time point. Next, the hematocrit value of the blood sample is calculated based on the two absorbances a₁ and a₂. The glucose concentration of the blood sample is then calculated based on the calculated hematocrit value and the absorbance a₂ for the second light λ₂ of the absorption wavelength of the pigment.

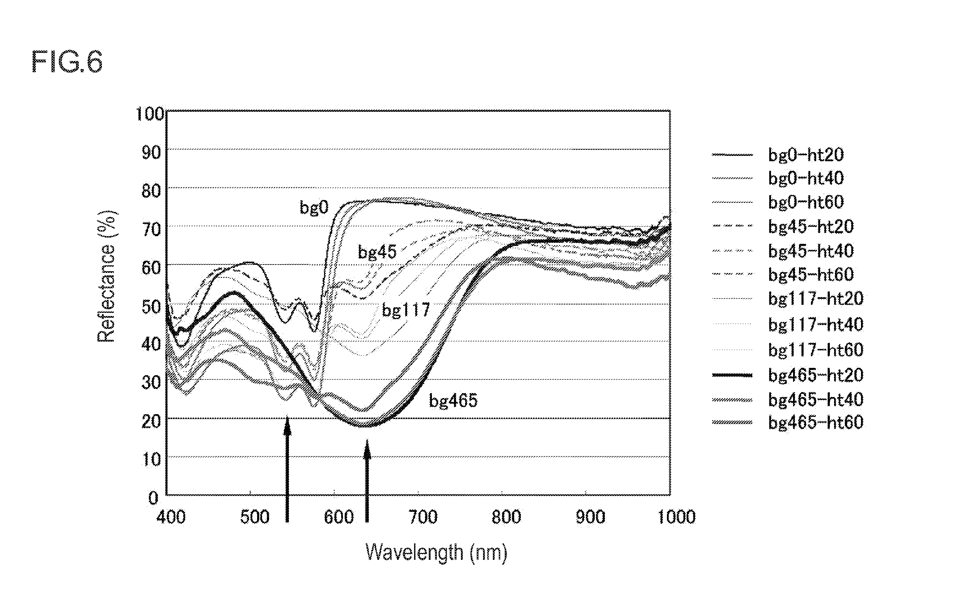

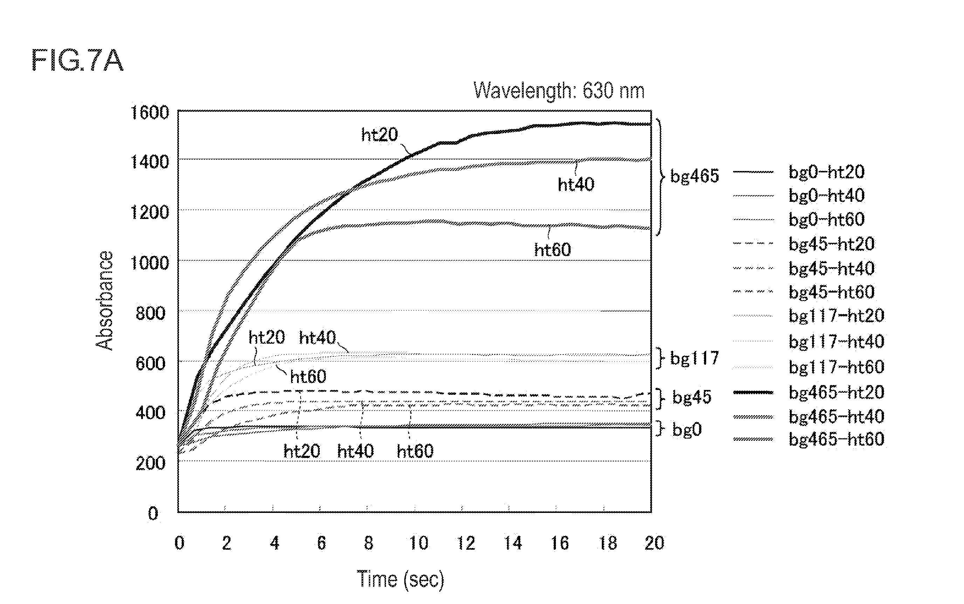

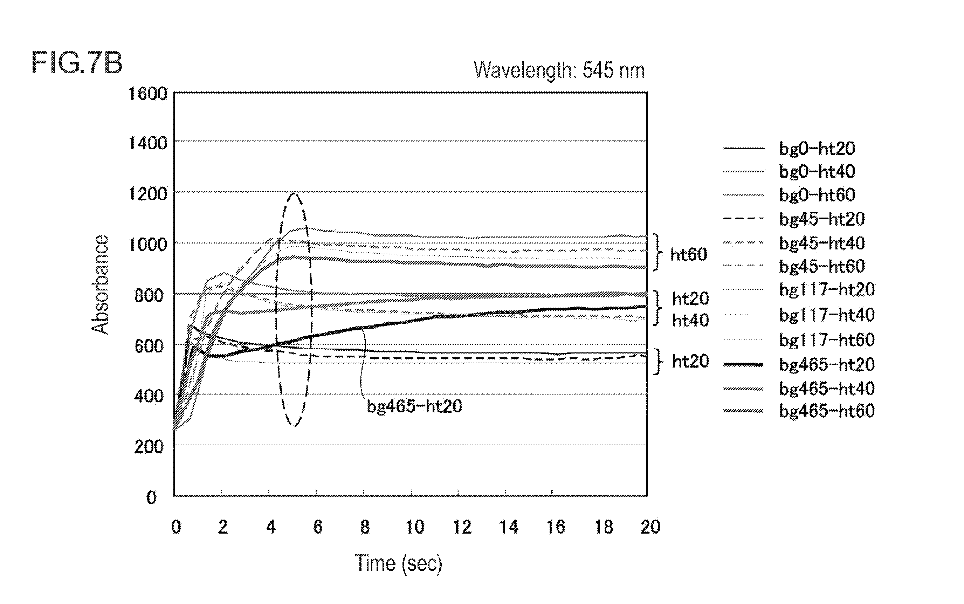

Next, the absorbance a₁ of the blood sample for the first light λ₁ of the absorption wavelength of the hemoglobin and the absorbance a₂ of the blood sample for the second light λ₂ of the absorption wavelength of the pigment related to chromogenic reaction of the blood sample will be described below with reference to Fig. 6 and Fig. 7.

Fig. 6 is a diagram showing coloring spectrum of the blood sample wherein glucose and hemoglobin are contained in various concentrations. The horizontal axis of Fig. 6 represents wavelength and the vertical axis represents the reflectance of the test paper on which the blood sample is absorbed. The reflectance of Fig. 6 is taken at the time point of 10 seconds after the coloration of the blood sample started by the chromogenic reagent that reacts with glucose. In the legend, bg45-ht20, for example, represents a blood sample adjusted to a blood glucose level of 45 mg/dl and a hematocrit value of 20%. As shown in Fig. 6, the wavelength/reflectance curves that show the relations between wavelength and reflectance for the blood samples containing glucose (bg45, bg117, bg465) reach the local minimal values in the vicinity of 630 nm. Therefore, we can see that it is preferable to use a light of a wavelength of approximately 630 nm as the second light λ₂ of the absorption wavelength of the pigment related to chromogenic reaction of the blood sample.

Also, the wavelength/reflectance curves that show the relations between wavelength and reflectance for the blood samples containing no glucose (bg0) reach the local minimal values in the vicinity of 545 nm and 575 nm. Therefore, we can see that it is preferable to use a light of a wavelength of approximately 545 nm or 575 nm as the first light λ₁ of the absorption wavelength of the hemoglobin.

Furthermore, as the coloring spectrum of such a first light λ₁ of the absorption wavelength of the hemoglobin tends to be affected by the coloring spectrum of the second light λ₂ of the absorption wavelength of the pigment, it is possible to eliminate its effect by using the second light λ₂ of the absorption wavelength of the pigment related to the chromogenic reaction of the blood sample in addition to the first light λ₁ of the absorption wavelength of the hemoglobin, so that the hematocrit value of the blood sample can be measured accurately.

Fig. 7A is a diagram showing a chronological change of the reflection absorbance relative to a light of the absorption wavelength of a pigment of a blood sample absorbed by a test paper, and Fig. 7B is a diagram showing a chronological change of the reflection absorbance relative to a light of the absorption wavelength of a hemoglobin when a blood sample is absorbed by a test paper. The horizontal axes of Fig. 7A and Fig. 7B represent time, and the vertical axes represent absorbance. The absorbance, i.e., the vertical axis, shows the reflection absorbance on the test paper, and represents 256/reflectance.

As shown in Fig. 7A, time/absorbance curves that indicate the relations between time and absorbance show the tendency that, the higher the glucose concentration (bg), the higher the absorbance a₂ of the blood sample for the second light λ₂ of the absorption wavelength of the pigment. Further, the absorbance a₂ of each blood sample reaches a saturation state at a time point approximately 10 seconds after the start of coloration so that it is suitable for calculating the blood glucose level.

On the other hand, as shown in Fig. 7B, the absorbance a₁ of the blood sample for the first light λ₁ of the absorption wavelength of the hemoglobin has a rising period in which the absorbance a₁ increases sharply as the time goes, and a stable period in which the absorbance a₁ varies more gradually than the change of the absorbance a₁ in the rising period. It is shown that there is a correlation, the higher the hematocrit value (ht), the higher the absorbance a₁, during a period immediately after a shift from the rising period to the stable period (for example, 5 seconds after the start of coloration). However, the correlation between the hematocrit value (ht) and the absorbance a₁ tends to disappear due to the influence of the pigment generated by chromogenic reaction as the time goes during the stable period. More specifically, the blood sample (bg465-ht20) with a glucose concentration of 465 mg/dl and a hematocrit value 20% shows the same absorbance a₁ as a blood sample with a hematocrit value 40%. Therefore, we can see that it is preferable to measure the absorbance a₁ of the blood sample for the first light λ₁ of the absorption wavelength of the hemoglobin at the time point of approximately 5 seconds after the start of coloration, i.e., the absorbance a₁ immediately after the shift from the rising period to the stable period in order to improve the measurement accuracy of the hematocrit value.

As can be seen from the above, the hematocrit value measuring method, the hematocrit value measuring device, the component measuring method, and the component measuring device of the present invention are described in the embodiment described above. Nonetheless, it goes without saying that the present invention can be added, modified, and simplified arbitrarily within its technological thought by a person skilled in the art.

For example, in the embodiment described above, a case of measuring glucose concentration using the component measuring device of the present invention was described. However, the intended use of the component measuring device of the present invention is not limited to the measurement of blood glucose level but can be applied for other components such as cholesterol. In this case, a chromogenic reagent is selected in accordance with the component to be measured and in response a specific light of an absorption wavelength specific to the pigment generated in the chromogenic reaction is selected.

Also, in the embodiment described above, a case of implementing the present invention was described using a simplified version of the component measuring device designed to do measurement by collecting the blood sample on the testing paper. However, the present invention is not limited to the particular device described in the above, it can also be materialized by various types of measuring devices, including those that measure the transmissive absorbance by collecting blood samples in a test tubes or cuvette.

Also, in the embodiment described above, the optical characteristic of the blood sample for a light of an absorption wavelength specific to a pigment was measured after measuring the optical characteristic of the blood sample for a light of an absorption wavelength specific to hemoglobin. However, the optical characteristic of the blood sample for a light of an absorption wavelength specific to a pigment and the optical characteristic of the blood sample for a light of an absorption wavelength specific to hemoglobin can be measured simultaneously.

### Examples

The embodiment of the present invention will be described in detail in the following with examples. However, the present invention shall not be construed to be limited to this examples in any way.

### <Comparative example>

Blood samples with known blood glucose level and hematocrit value were prepared. The blood glucose levels of the prepared blood samples were 0 mg/dl, 45 mg/dl, 117 mg/dl, and 465 mg/dl, while hematocrit values were 20%, 40%, and 60%.

Also, the tips using a polyethersulfone membrane which was impregnated with a chromogenic reagent and was dried for 30 minutes at 40 degrees C with a thickness of approximately 150 µm as a test paper, were prepared. The chromogenic reagent had a composition of glucose oxidase (150 mg), peroxidase (75 mg), 4-aminoantipyrine (10 mg), N-ethyl-N-(2-hydroxy-3-sulfopropyl)-m-toluidine (20 mg), and 0.2 M phosphate buffer solution (pH 6) (15 ml). The material of the fine tube part of the tip, through which the blood sample is introduced by capillary action, was acrylic resin and its inner diameter was 0.35 mm.

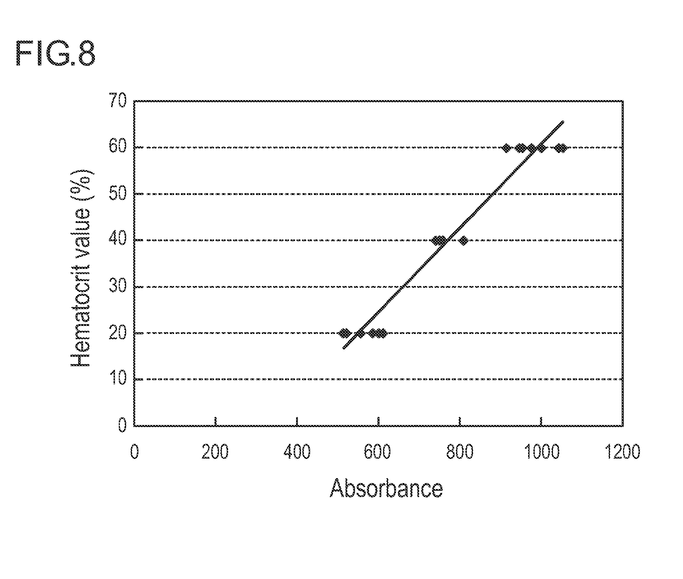

More than 5 µl of blood sample was introduced into the holder via the fine tube part of the tip to be absorbed on the back surface of the test paper and the front surface of the test paper was irradiated with light of an absorption wavelength (545 nm) specific to hemoglobin in order to measure the reflectance of the test paper 5 seconds after the start of coloration. For measurement of the reflectance, a colorimeter (Nihon Bunko-sha, Sigma 80) was used. The hematocrit value of each blood sample was calculated based on the measured reflectance. In the present comparative example, as shown in Fig. 8, the conversion table showing the relation between the absorbance of the blood sample for the light of the absorption wavelength of the hemoglobin and the hematocrit value was a polynomial equation of the fist order (approximate straight line) calculated by the least square method. The result of the measurement is shown in Table 1.

**[Table 1]**

| Blood glucose level (mg/dl) | Actual Ht (%) | Calculated Ht (%) | Calculated error |
|---|---|---|---|
| 0 | 20 | 23.6 | 3.6 |
| 0 | 40 | 43.6 | 3.6 |
| 0 | 60 | 64.9 | 4.9 |
| 45 | 20 | 20.9 | 0.9 |
| 45 | 20 | 20.7 | 0.7 |
| 45 | 40 | 39.0 | -1.0 |
| 45 | 40 | 38.3 | -1.7 |
| 45 | 60 | 61.2 | 1.2 |
| 45 | 60 | 65.9 | 5.9 |
| 117 | 20 | 17.0 | -3.0 |
| 117 | 20 | 17.7 | -2.3 |
| 117 | 40 | 37.9 | -2.1 |
| 117 | 40 | 37.6 | -2.4 |
| 117 | 60 | 56.9 | -3.1 |
| 117 | 60 | 59.0 | -1.0 |
| 465 | 20 | 24.9 | 4.9 |
| 465 | 20 | 25.7 | 5.7 |
| 465 | 40 | 37.8 | -2.2 |
| 465 | 40 | 37.2 | -2.8 |
| 465 | 60 | 53.7 | -6.3 |
| 465 | 60 | 56.4 | -3.6 |
| Standard deviation of calculated errors | | | 3.53 |

As shown in Table 1, there were errors between the actual hematocrit values (actual Ht) and the hematocrit values obtained by measurement and calculation (calculated Ht). The standard deviation of the errors between the actual hematocrit values and the measured hematocrit values was 3.53.

### <Example 1>

In the example 1, under the same condition as the comparative example, varieties of blood samples were absorbed by the test paper and irradiated with the light of the absorption wavelength (545 nm) specific to hemoglobin, and the light of the absorption wavelength (630 nm) specific to the pigment related to the chromogenic reaction of the blood samples in order to measure the reflectance of the test paper 10 seconds after the start of coloration. The hematocrit value was then calculated based on the two measured reflectances. The result of the measurement is shown in Table 2.

**[Table 2]**

| Blood glucose level (mg/dl) | Actual Ht (%) | Calculated Ht (%) | Calculated error |
|---|---|---|---|
| 0 | 20 | 20.8 | -0.8 |
| 0 | 40 | 40.6 | -0.6 |
| 0 | 60 | 57.4 | 2.6 |
| 45 | 20 | 20.0 | 0.0 |
| 45 | 20 | 20.6 | -0.6 |
| 45 | 40 | 40.1 | -0.1 |
| 45 | 40 | 38.9 | 1.1 |
| 45 | 60 | 58.9 | 1.1 |
| 45 | 60 | 62.8 | -2.8 |
| 117 | 20 | 18.5 | 1.5 |
| 117 | 20 | 19.6 | 0.4 |
| 117 | 40 | 40.9 | -0.9 |
| 117 | 40 | 40.2 | -0.2 |
| 117 | 60 | 58.7 | 1.3 |
| 117 | 60 | 62.0 | -2.0 |
| 465 | 20 | 22.1 | -2.1 |
| 465 | 20 | 21.9 | -1.9 |
| 465 | 40 | 37.7 | 2.3 |
| 465 | 40 | 37.1 | 2.9 |
| 465 | 60 | 59.6 | 0.4 |
| 465 | 60 | 61.5 | -1.5 |
| Standard deviation of calculated errors | | | 1.60 |

As shown in Table 2, the standard deviation of the errors between the actual hematocrit values (actual Ht) and the hematocrit values obtained by measurement and calculation (calculated Ht) was 1.60. Therefore, it was confirmed that the hematocrit value can be accurately measured by means of using two different kinds of light of different wavelengths.

### <Example 2>

In the example 2, under the same condition as the comparative example, varieties of blood samples were absorbed by the test paper and irradiated with the light of the absorption wavelength (545 nm) specific to hemoglobin in order to measure the reflectance of the test paper 5 seconds after the start of coloration. Next, the light of the absorption wavelength (630 nm) specific to the pigment was radiated to measure the reflectance of the test paper 10 seconds after the start of coloration. The hematocrit value was then calculated based on the two measured reflectances. The result of the measurement is shown in Table 3.

**[Table 3]**

| Blood glucose level (mg/dl) | Actual Ht (%) | Calculated Ht (%) | Calculated error |
|---|---|---|---|
| 0 | 20 | 20.5 | -0.5 |
| 0 | 40 | 40.6 | -0.6 |
| 0 | 60 | 58.0 | 2.0 |
| 45 | 20 | 20.8 | -0.8 |
| 45 | 20 | 20.1 | -0.1 |
| 45 | 40 | 39.8 | 0.2 |
| 45 | 40 | 39.1 | 0.9 |
| 45 | 60 | 58.9 | 1.1 |
| 45 | 60 | 62.6 | -2.6 |
| 117 | 20 | 18.2 | 1.8 |
| 117 | 20 | 19.3 | 0.7 |
| 117 | 40 | 41.3 | -1.3 |
| 117 | 40 | 41.2 | -1.2 |
| 117 | 60 | 58.9 | 1.1 |
| 117 | 60 | 60.6 | -0.6 |
| 465 | 20 | 21.5 | -1.5 |
| 465 | 20 | 21.3 | -1.3 |
| 465 | 40 | 38.3 | 1.7 |
| 465 | 40 | 37.9 | 2.1 |
| 465 | 60 | 59.4 | 0.6 |
| 465 | 60 | 61.7 | -1.7 |
| Standard deviation of calculated errors | | | 1.35 |

As shown in Table 3, the standard deviation of the errors between the actual hematocrit values (actual Ht) and the hematocrit values obtained by measurement and calculation (calculated Ht) was 1.35. Therefore, it was confirmed that the hematocrit value can be more accurately measured by means of measuring the absorbance for light of a wavelength of 545 nm at an earlier time point than the absorbance for light of a wavelength of 630 nm in addition to using two different kinds of light of different wavelengths.

The present application is based on the Japanese patent application 2008-078880 filed on March 25, 2008, the contents of which are cited herein in its totality to be a part of this application.

### Explanation of numerals

- 10: Component measuring device
- 20: Measuring device main body
- 21: Light measuring unit
- 25: Light emitting element
- 26: Light receiving element
- 30: Tip
- 32: Test paper
- 40: Control unit
- 41: CPU
- 42: Data memory unit

## Claims

1. A method of measuring hematocrit value of a blood sample in a chromogenic reaction using a chromogenic reagent that reacts with a blood component that is different from hemoglobin, **characterized by**:
calculating the hematocrit value of said blood sample based on an optical characteristic of said blood sample measured with a light of at least one absorption wavelength specific to hemoglobin and an optical characteristic of said blood sample measured with a light of at least one absorption wavelength specific to a pigment generated in said chromogenic reaction.

2. The method of measuring hematocrit value according to claim 1, **characterized in that** said optical characteristic of the blood sample relative to said light of the absorption wavelength of hemoglobin is measured at an earlier time point than said optical characteristic of the blood sample relative to said light of the absorption wavelength of said pigment.

3. A method of measuring concentration of a blood component that is different from hemoglobin by means of a chromogenic reaction using a chromogenic reagent that reacts with said blood component, **characterized by** comprising:
a step of irradiating a blood sample with a light of at least one absorption wavelength specific to hemoglobin to measure a first optical characteristic of said blood sample relative to said light of the absorption wavelength of hemoglobin;
a step of irradiating said blood sample with a light of at least one absorption wavelength specific to a pigment generated in said chromogenic reaction to measure a second optical characteristic of said blood sample relative to said light of the absorption wavelength of said pigment;
a step of calculating the hematocrit value of said blood sample based on said first optical characteristic and said second optical characteristic; and
a step of calculating the concentration of said component of said blood sample based on said calculated hematocrit value and said second optical characteristic.

4. A device for measuring hematocrit value of a blood sample in a chromogenic reaction using a chromogenic reagent that reacts with a blood component that is different from hemoglobin, **characterized by**:
calculating the hematocrit value of said blood sample based on an optical characteristic of said blood sample measured with a light of at least one absorption wavelength specific to hemoglobin and an optical characteristic of said blood sample measured with a light of at least one absorption wavelength specific to a pigment generated in said chromogenic reaction.

5. A device for measuring concentration of a blood component that is different from hemoglobin by means of a chromogenic reaction using a chromogenic reagent that reacts with said blood component, **characterized by** comprising:
first light emitting means for irradiating a blood sample with a light of at least one absorption wavelength specific to hemoglobin;
fist light receiving means for receiving the light that is radiated from said first light emitting means and is reflected by said blood sample or passes through said blood sample to measure a first optical characteristic of said blood sample relative to said light of the absorption wavelength of hemoglobin;
second light emitting means for irradiating said blood sample with a light of at least one absorption wavelength specific to a pigment generated in said chromogenic reaction;
second light receiving means for receiving the light that is radiated from said second light emitting means and is reflected by said blood sample or passes through said blood sample to measure a second optical characteristic of said blood sample relative to said light of the absorption wavelength of said pigment;
hematocrit value calculation means for calculating the hematocrit value of said blood sample based on said first optical characteristic and said second optical characteristic; and
component concentration calculation means for calculating the concentration of said component of said blood sample based on said calculated hematocrit value and said second optical characteristic.
